Europäisches Patentamt

European Patent Office

Office européen des brevets

(19)

(11) Veröffentlichungsnummer: **0 155 237**
**B1**

(12) **EUROPÄISCHE PATENTSCHRIFT**

(45) Veröffentlichungstag der Patentschrift:
**31.05.89**

(51) Int. Cl.⁴: **C 12 M 3/02, C 12 M 1/12**

(21) Anmeldenummer: **85810103.3**

(22) Anmeldetag: **11.03.85**

(54) **Verfahren und Vorrichtung zum Kultivieren von humanen, tierischen, pflanzlichen sowie hybriden Zellen und Mikroorganismen.**

(30) Priorität: **15.03.84 DE 3409501**

(43) Veröffentlichungstag der Anmeldung:
**18.09.85 Patentblatt 85/38**

(45) Bekanntmachung des Hinweises auf die Patenterteilung:
**31.05.89 Patentblatt 89/22**

(84) Benannte Vertragsstaaten:
**AT BE CH DE FR GB IT LI LU NL SE**

(56) Entgegenhaltungen:
**EP-A-0 112 155**
**DE-C-839 245**
**FR-A-2 324 365**
**FR-A-2 393 849**
**US-A-3 186 917**

(73) Patentinhaber: **MBR Bio Reactor AG, Werkstrasse 3/4, CH- 8620 Wetzikon (CH)**

(72) Erfinder: **Katinger, Hermann, Dr., Heiligenstädterstrasse 133, A-1190 Wien (AT)**
Erfinder: **Scheirer, Winfrid, Gartengasse 8, A-2351 Wiener Neudorf (AT)**

(74) Vertreter: **Herrmann, Peter Johannes, c/o PPS Polyvalent Patent Service AG Mellingerstrasse 1, CH- 5400 Baden (CH)**

EP 0 155 237 B1

**Beschreibung**

Die Erfindung betrifft ein Verfahren und eine Vorrichtung zum Kultivieren von humanen, tierischen, pflanzlichen sowie hybriden Zellen und Mikroorganismen in einem Bioreaktor mit wenigstens drei aneinander anliegenden Kammern, wobei wenigstens eine erste Kammer eine Zellzuchtkammer, eine zweite Kammer eine Mediumkammer und eine dritte Kammer eine Produktkammer ist und die einzelnen Kammern voneinander durch Membranen begrenzt sind.

Zellen höherer Eukaryonten (tierische Zellen, humane Zellen, Pflanzenzellen und Mikroorganismen) sind in vitro durch folgende Eigenschaften charakterisiert:
- Wachstum in komplexen Nährmedien.
- Hohe Empfindlichkeit gegenüber mechanischen und hydraulischen Schereffekten.
- Hohe Empfindlichkeit gegenüber physikalischen und chemischen Umwelteinflüssen.
- Weitgehende Abhängigkeit von einer ausgeglichenen Versorgung mit Nährstoffen und Entsorgung der Stoffwechselprodukte.
- Die Kinetik der Produktbildung kann sowohl an das Zellwachstum gebunden sein als auch unabhängig davon erfolgen.
- Die Produkte werden entweder ausgeschieden oder sind zellassoziiert.

Bei der Züchtung von Zellen werden diese mit Nährstoffen aus dem sie umgebenden Nährmedium versorgt. Die Stoffwechselprodukte der Zellen werden in das Medium abgegeben. Wenn das die Zellen enthaltende flüssige Nährmedium in einem Behälter ruhig steht, so setzen sich die Zellen wegen ihres grösseren spezifischen Gewichtes am Behälterboden ab und/oder lagern sich an den Behälterwänden an. Die sich daraus ergebenden hohen Zelldichten haben zur Folge, dass die Diffusion zum Nährstoffnachschub bzw. zur Entsorgung von Stoffwechselprodukten nicht ausreicht und die Zellen absterben. Darüber hinaus ist zur Erlangung von hohen Zelldichten, wie sie bei Produktionsprozessen vielfach gewünscht werden, ein Nachschub und/oder ein Austausch bzw. eine Regenerierung des Mediums notwendig. Üblicherweise muss daher die Zellsuspension entweder in sehr dünnen Schichten kultiviert werden oder für eine ständige Aufrechterhaltung einer Suspension gesorgt werden, bzw. müssen die Zellen beim Mediumnachschub oder Mediumtausch kurzfristig vom Medium separiert werden.

In der FR-A-2 393 849 ist ein Verfahren und eine Vorrichtung der eingangs beschriebenen Art enthalten. Die Lösung gemäss dieser Patentanmeldung weist jedoch den Nachteil auf, dass alle Zellen mit einer verhältnismässig starken Strömung belastet werden, so dass die mechanische Abscherung der Zellen und somit ihre Beschädigung in Frage kommt. Die Probleme mit der mechanischen und chemischen Belastbarkeit der Zellen gilt selbstverständlich nicht nur bei Verfahren und Vorrichtung der genannten Art, sondern auch zum Beispiel beim Rühren, Füllen, Zentrifugieren, Filtrieren, Sterilhalten usw. Bei der Bearbeitung im Labormaßstab ist die Gefahr der Beschädigung der Zellen verhältnismässig klein. Bei industriellen, grosstechnischen Anlagen erhöht sich diese Gefahr wesentlich und es sind also Massnahmen notwendig, die die Beschädigung der Zellen verhindern oder wenigstens vermindern.

Aufgabe der Erfindung ist es, unter weitgehend natürlichen Bedingungen, die Zufuhr von Nährstoffen und die Abfuhr von Stoffwechselprodukten unter Vermeidung von mechanischen und chemischen Einflüssen zu erzielen.

Die vorgenannte Aufgabe wird erfindungsgemäss dadurch gelöst, dass die Zellen innerhalb der Zellzuchtkammer an Trägern aus netzartigen Geweben immobilisiert werden, die die Membranen mechanisch unterstützen und dass deren rahmenartige Ränder als Dichtung und gleichzeitig als Distanzkörper dienen, wobei eine mit einem sauerstoffhaltigen Gas angereicherte Nährlösung enthaltende Mediumkammer in ihrer Längsrichtung kontinuierlich durchströmt wird und dass das Produkt aus der Zellzuchtkammer nur diskontinuierlich abgezogen wird.

Die Zellen werden in einer Zellzuchtkammer kultiviert, deren Raum durch die beiden gegenüberliegenden Membranen und durch zusätzliche Haltevorrichtungen, Anschlüsse usw. begrenzt wird. Die Kammern sind sehr dünn und die Zellen ruhen in ihnen, was eine mechanische Abscherung der Zellen verhindert. Die Produktkammern werden je nach einer längeren Zeit, z. B. nach 24 Stunden, abgezogen, so dass die Gefahr der Abscherung durch ständige Strömung wesentlich vermindert wird. Ein weiterer Vorteil des Verfahrens ist darin zu sehen, dass die Diffusion durch die Membranen zwischen Mediumkammern und Zellzuchtkammern entsprechend der Wahl der Membranen realisiert wird und dass die Membranen mit dem netzartigen Gewebe unterstützt werden und auf einfache Weise gemeinsam mit dem genannten Gewebe an Rändern befestigt und durch Zusammendrücken auch abgedichtet werden.

Die Vorrichtung zur Durchführung des Verfahrens ist so ausgebildet, dass wenigstens eine Zellzuchtkammer über eine, auf einem netzartigen Gewebe abgestützte Membran mit einer Mediumkammer in Verbindung steht, dass die erste Membran eine obere Ausschlussgrenze von 100'000 Dalton aufweist und das Produkt vom Nährmedium trennt, dass die Zellzuchtkammer über eine zweite, auf einem anderen netzartigen Gewebe abgestützte Membran an die Produktkammer angeschlossen ist, wobei die zweite Membran (B) eine Porosität aufweist, die den Abzug des Produktes aus der Zellzuchtkammer unter Verhinderung des Abzugs der Zellen erlaubt. Die Nährmediumzufuhr erfolgt vorzugsweise durch die Poren der Membrane entweder durch Diffusion oder durch Massenfluss. Ebenso können Produkte der Zellen durch die Membranen abgeführt aber auch - wählbar durch die Durchlässigkeit der Membran - im Kulturraum zurückgehalten werden. Nach einer zweckmässigen Ausführungsform weist die erste Membran eine Ausschlussgrenze von 100'000 Dalton auf. Diese Dimensionierung hat sich in praxi bewährt und die Teilchen, die 100'000 Dalton übersteigen, können nicht mehr durch die Membran passieren.

Zweckmässig weist die Porengrösse der zweiten Membran 0,1 µm bis 0,6 µm, vorzugsweise 0,2 µm, auf. Es ist selbstverständlich, dass auch kleinere Abweichungen von dieser Porengrösse sehr gute Dienste leisten können.

Es ist vorteilhaft, wenn die erste und die zweite Membran in der Zellzuchtkammer einen Abstand von 0,3 bis 10 mm aufweisen. Es hat sich als besonders vorteilhaft erwiesen, den Abstand beider Membranen mit 0,5 mm zu wählen, da die als Träger für die Zellen dienenden Netze auch 0,5 mm dick sind und so mit ihrer Textur die ganze Zellzuchtkammer ausfüllen.

Nach einer Weiterbildung ist es zweckmässig, wenn eine Mess-Sonde durch einen orthogonal durch die Deiden an die Zellzuchtkammer anliegenden Kammern hindurchführenden Anschlußstutzen und eine weitere Mess-Sonde durch einen Anschlußstutzen mit dem Messkopf innerhalb der Zellzuchtkammer fixiert ist. Diese Leitungen führen rechtwinklig durch alle Kammern hindurch, wobei der Kopf eines Messfühlers etwa in der Mitte einer Zellzuchtkammer fixiert ist. Es sind insbesondere Sonden für pH, pO$_2$ vorgesehen.

Als besonders vorteilhaft zum Immobilisieren von Zellen, haben sich als Träger Gewebe erwiesen, welche eine netzartige Textur aufweisen und aus Fluorkohlenwasserstoffen bestehen.

Zweckmässig sind die Netze in ihren Rahmen schräg angeordnet. Hierbei setzen sich die Zellen um die Gewebefäden fest und lassen eine Öffnung in der Mitte für den Stoffaustausch frei. Die Rahmen dienen gleichzeitig als Dichtungs- und Distanzierungskörper.

Die Kulturkammern können als Stapel mit gemeinsamen Zu- und Ablaufkanälen aufgebaut werden, wobei ein Dreikammersystem (Medium/Zellen/Produkt) als minimale funktionelle Einheit angewendet werden kann. Die Anzahl der Kammern in einem Stapel kann jedoch beliebig gewählt werden. Die Fläche der Kulturkammern ist durch die produktionsbedingte Maximalgrösse der Membranen begrenzt.

In einer Zellzuchtkammer können zwei Membranen mit unterschiedlicher Porengrösse verwendet werden. Dabei ist die Porengrösse der Membran zwischen Zellen und Nährmedium so zu wählen, dass ein Nährstofftausch stattfinden kann und die Membran zwischen den Zellen und der Produktekammer, dass lediglich die Stoffwechselprodukte auf die Produktseite hindurchtreten können.

Die Membranen können aus jedem zu diesem Zweck brauchbaren Material bestehen und jede brauchbare Porengrösse aufweisen. Sie können symmetrisch oder asymmetrisch, polar oder unpolar, hydrophil oder hydrophob sein und müssen in ihrer Stärke dem System angepasst werden. Die Membranen müssen jedoch so beschaffen sein, dass im wesentlichen die Zellen zurückgehalten werden. Es gelten die genannten Kriterien auch für gaspermeable Membranen. Die Höhe der Kammer zwischen den bevorzugt horizontal liegenden Membranen ist durch die Nährmediumzufuhr und die gewünschten Zelldichten begrenzt. Die Kulturkammer kann mit Einbauten zum Stützen der Membranen und/oder zur Erzeugung von Konvektion und/oder zur kontrollierten Leitung der Suspension versehen werden. Die Mediumkammern sind in ihren Volumina nur durch den gewünschten Durchfluss bzw. durch die Leiteinrichtungen zur Steuerung der Konvektion und der Strömung sowie das gewünschte Volumen begrenzt.

Der Gesamtaustausch (Ver- und Entsorgung) von Gasen, wie O$_2$, CO$_2$ usw., kann entweder durch externe oder interne Anreicherung im Kulturmedium in gelöster Form oder als Gas/Flüssigkeits-Dispersion erfolgen, aber auch durch gaspermeable Membranen beliebiger Form durchgeführt werden. Dies kann entweder analog zu den Medienströmen oder durch Einsatzmembranen, z. B. schlauchförmige Membranen, die durch die Kulturkammern geführt werden und gleichzeitig als Stütznetze der Flachmembranen und/oder Träger der Zellen dienen, erfolgen.

Die Erfindung wird anhand von einigen Beispielen näher erläutert.

Es zeigen:

Fig. 1    ein Prinzipschema des Verfahrens mit einem Aufriss durch die Vorrichtung gemäss der Erfindung,

Fig. 2    den Grundriss der Zellzuchtvorrrichtung gemäss Fig. 1,

Fig. 3    den Schnitt A-A aus der Fig. 2,

Fig. 4    den Schnitt B-B aus der Fig. 2,

Fig. 5    eine Anordnung der Mess-Sonden in der Zellzuchtkammer,

Fig. 6    eine Vergrösserung im Bereich der Befestigung der Membranen,

Fig. 7    eine perspektivische Ansicht auf den Aufbau der Zellzuchtvorrichtung

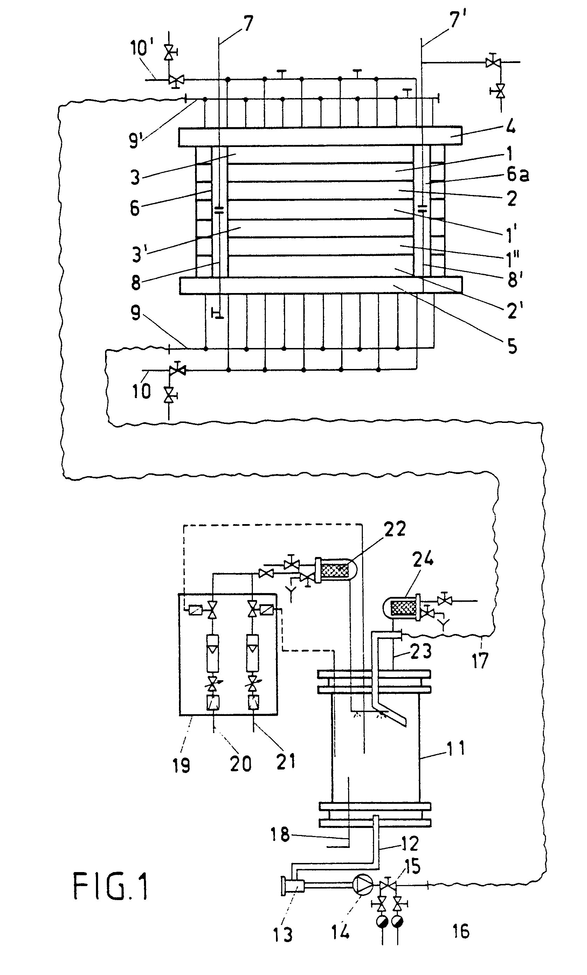

Gemäss Fig. 1 besteht eine Zellzuchtvorrichtung in ihrer einfachen Ausführung aus je einer Zellzuchtkammer 1, welche von einer Mediumkammer 2, und einer Produktkammer 3 eingeschlossen wird. In Fig. 1 sind weitere Zellzuchtkammern 1', 1", eine weitere Mediumkammer 2' und eine weitere Produktkammer 3' gezeigt. Die Vorrichtung ist je nach den Bedürfnissen beliebig erweiterungsfähig. Die drei Kammern werden durch eine Deckplatte 4 und eine Bodenplatte 5 begrenzt. Die einzelnen Kammern sind durch Membranen und an diesen randseitig angebrachten Dichtungen steril abgedichtet. In einer Leitung 6 zur Zellenzufuhr und Zellenernte sind Sonden 7 bzw. 7' zur Messung bzw. Steuerung von pH und pO$_2$ vorgesehen. Zur Fixierung der Sonden innerhalb beispielsweise in der Zellzuchtkammer 1' sind Feststellbolzen 8 bzw. 8' vorgesehen. Die Zellzuchtvorrichtung ist mit Anschlüssen 9, 9' für ein Nährmedium sowie mit Anschlüssen 10, 10' für das Produkt versehen. Ein Behälter 11 zur Aufbereitung des Nährmediums ist über eine Leitung 12, ein Dosierventil 13, eine Pumpe 14, ein Ventil 15 und eine flexible Leitung 16 mit dem Anschluss 9 an der Zellzuchtvorrichtung verbunden. Über eine Leitung 17 wird das Medium in den Behälter 11 zurückgeführt. Der Behälter 11 ist mit einer Temperaturregelung 18 verbunden sowie einer Begasungseinrichtung 19. Die Begasungseinrichtung 19 beinhaltet eine Zufuhrleitung 20 für Luft und eine Zufuhrleitung 21 für Kohlenstoffdioxid. Zwischen der Begasungsstation 19 und dem Behälter 11 ist ein Zuluftfilter 22 und in der Abluftleitung 23 ein Abluftfilter 24 vorgesehen.

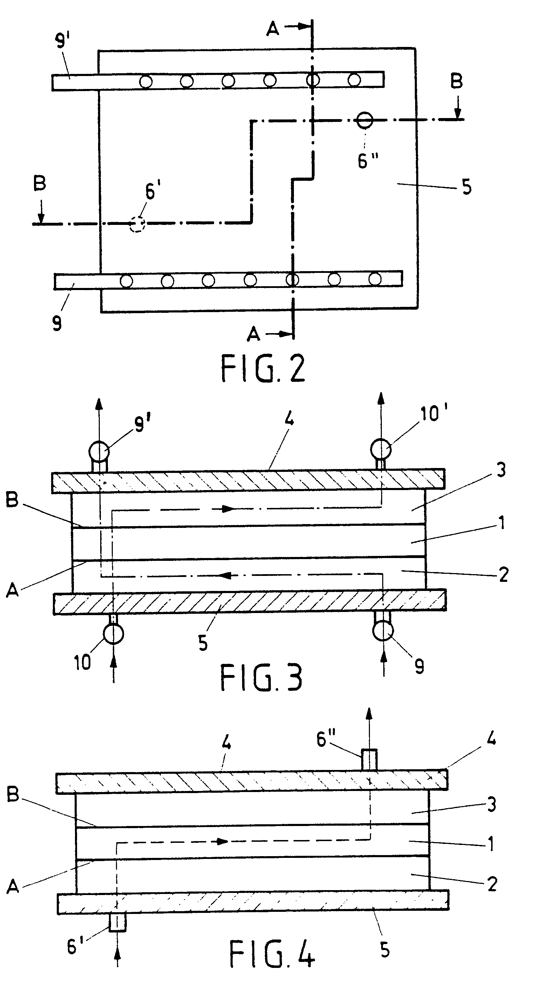

Fig. 2 zeigt den Grundriss der Zellzuchtvorrichtung gemäss Fig. 1 mit der Bodenplatte 5, dem Anschluss 9 für die Mediumzufuhr und dem Anschluss 9' für die Mediumabfuhr von und zu den Mediumkammern 2 und 2' sowie die Anschlüsse 6' und 6" zu den Zellzuchtkammern 1, 1', 1".

In Fig. 3 ist gemäss dem Schnitt A-A aus der Fig. 2 die Zellzuchtkammer 1, die Mediumkammer 2 und die Produktkammer 3 zwischen der Deckplatte 4 und der Bodenplatte 5 befestigt. Zwischen der Zellzuchtkammer 1 und der Mediumkammer 2 ist eine Membran A und zwischen der Produktkammer 3 eine weitere Membran B vorgesehen. Die Mediumzufuhr erfolgt über den Anschluss 9 und die Mediumabfuhr über den Anschluss 9'. Der Anschluss 10 ist für den Produkteintritt und der Anschluss 10' für den Produktaustritt vorgesehen. Sowohl der Mediumstrom als auch der Produktstrom sind durch mit Pfeilen vorgesehenen punktgestrichelten Linien angedeutet.

In Fig. 4 (Schnitt B-B, Fig. 2) ist der Anschluss 6' für den Eintritt der Zellsuspension und der Anschluss 6" für den Austritt der Zellsuspension vorgesehen. Hier ist der Weg der Zellsuspension gleichfalls durch eine strich-punktierte Linie wiedergegeben.

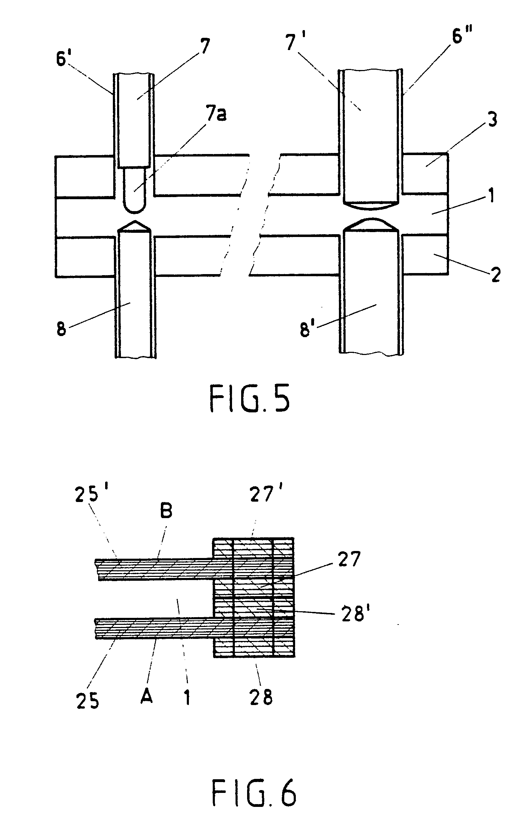

In Fig. 5 ist die Anordnung von Sonden 7, 7' im Eintrittsstutzen 6' und Austrittsstutzen 6" der Zellzuchtkammer 1 gezeigt, die zur Fixierung in ihrer Eindringtiefe von den Feststellbolzen 8 und 8' begrenzt sind. Ein Messkopf 7a befindet sich in der Mitte der Zellzuchtkammer 1.

In Fig. 6 ist die Befestigung der Membranen A, B und von Geweben 25, 25', welche zur Immobilisierung der Zellen oder Mikroorganismen dienen, gezeigt. Die Befestigung erfolgt durch Zusammenklemmen an der Peripherie, wobei auf den Geweben rahmenartig aufgebrachtes Dichtungsmaterial 27, 27' und 28, 28' gleichzeitig als Distanzhalterung dient.

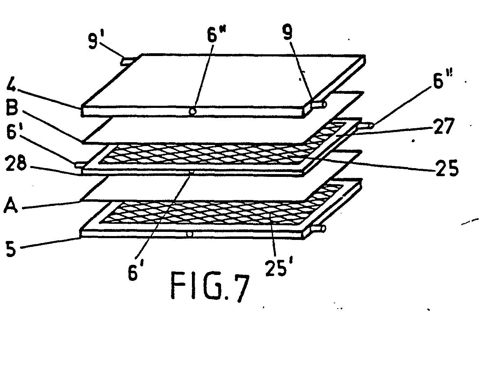

Fig. 7 zeigt eine geöffnete Zellzuchtkammer mit der Deckplatte 4 und der Bodenplatte 5. Zwischen der ersten Membrane A und der zweiten Membrane B sind Gewebe 25 mit der erfindungsgemässen Textur versehen, die gleichzeitig als Stützgewebe für die Membranen und als Träger 25 bzw. 25' für die Zellen dienen. Die schräge Anordnung der Textur des netzartigen Gewebes 25, 25' in ihrem Rahmen ist in der Fig. 7 dargestellt.

Die Betriebsweise wird anhand von Fig. 1 erläutert. Nach dem Zusammenbau einer Zellzuchtvorrichtung bestehend aus den Zellzuchtkammern 1, 1', 1", den Mediumkammern 2, 2', den Produktkammern 3, 3' sowie der Deckplatte 4 und der Bodenplatte 5, den Membranen A und B sowie den in den Zellzuchtkammern 1, 1', 1" zwischen den Membranen befestigten Netzen (Fig. 7), wird die Apparatur auf Dichtigkeit geprüft und in bekannter Weise mit Dampf sterilisiert. Zur Immobilisierung der Zellen wird eine Zellsuspension über die Leitung 6 der linken Seite die Zellzuchtkammern 1, 1', 1" und die Ausgangsleitung 6a auf der rechten Seite der Vorrichtung über ein entprechendes nicht gezeigtes Impfgefäss so lange zirkuliert, bis alle Zellen auf den Netzen fixiert sind.

Gleichzeitig wird eine im Behälter 11 bereitete sterile Nährlösung als Medium mittels der Pumpe 14, über die Leitung 16, den Anschluss 9, die Mediumkammern 2 und 2', die Sammelleitung 9', Leitung 17 in den Behälter 11 rezirkuliert. Die Menge des Medienstroms wird mit dem Ventil 13 eingestellt. Die Begasung des Nährmediums erfolgt mittels der Begasungseinrichtung 19, welche eine Dosiereinheit für die Zugabe von Luft über die Leitung 20 und CO$_2$ über die Leitung 21 vorsieht. Diese Gase werden vor dem Eintritt in den Behälter 11 über das Zuluftfilter 22 sterilfiltriert. Die Abgase aus dem Behälter 11 können über das Abluftfilter 24 entweichen.

Über den Anschluss 10 kann produktseitig eine geeignete Flüssigkeit beispielsweise für den Abtransport der Stoffwechselprodukte aus den Zellzuchtkammern 1, 1', 1" geleitet werden. Diese durchströmt z. B. nach 24 Stunden die Produktekammern 3 und 3' und verlässt die Zellzuchtvorrichtung über den Anschluss 10'. Somit wird das Produkt diskontinuierlich abgezogen, wobei während des Zellzuchtbetriebs die Ventile zu den Anschlüssen 10 und 10' geschlossen bleiben, bis sich durch die Permeation durch die Membranen in den Produktskammern 3 und 3' genügend Produkt angesammelt hat, welches unter Druck steht und über die Leitung 10' im Bedarfs- bzw. Erntefall abgezogen werden kann.

Die Betriebsweise der Kulturkammmern lässt sich auf die jeweilige Produktionskinetik optimal abstimmen. Dies wird dadurch gewährleistet, dass eine Immobilisierung der Zellen in einem gewünschten Ausmass erreicht wird und sowohl die Zellseite als auch die Mediumseite getrennt kontinuierlich oder diskontinuierlich durchströmt werden können. Die Vorteile der bekannten Verfahren der Zellimmobilisierung, der Dialysekultur und der Perfusionskultur können mit der Methode der diskontinuierlichen Betriebsweise dadurch kombiniert werden. Es kann das erfindungsgemässe Verfahren für biokatalytische Umwandlungen von Substraten zu Produkten verwendet werden. Das erfindungsgemässe Verfahren ergibt eine leichtere Bearbeitbarkeit und bessere Kontrollierbarkeit sowie die Möglichkeit der kontrollierten Durchströmung und der Messwerterfassung im Kulturraum. Im Vergleich zum komplizierten Verfahren der Mikroverkapselungstechnik gewährleisten weit einfachere und kleinere Anlagen eine bessere Kontrollierbarkeit und liefern dabei gleich gute oder bessere Ergebnisse.

**Beispiel**

Die Kulturkammern werden mit Membranen aus Polysulfon von 0,2 μm Porengrössen bestückt, die Zu- und Ablaufkanäle von Kultur- und Mediumkammern parallel zusammengefasst und der Apparat sterilisiert.

Die Kulturkammern werden mit einer Zellsuspension der Hybridom-Zellinie C28 befüllt. Es ist dies eine Hybridomzellinie zwischen primären Humanzellen und einer Mäusemyeolomzellinie. Sie produziert humanes IgGI und scheidet dieses in das umgebende Medium aus. Dieses Kulturmedium besteht aus 90 % Dulbecco's MEM und 10 % foetalem Kalbserum. Die Startzelldichte beträgt 500'000 Zellen/ml. Die Kultivierungstemperatur ist 36,5° C. In den Mediumkanälen wird Kulturmedium, das mittels einer Airliftpumpe mit Luft angereichert wird, umgepumpt und mit einem Volumenäquivalent des Kulturkamm ervolumens täglich durchströmt. Nach drei Tagen ist eine Zelldichte von $1,8 \times 10^6$ Zellen/ml erreicht, die IgGI-Konzentration auf der Mediumseite beträgt etwa 30 mg/l. Die Globulinkonzentration steigt allmählich auf etwa 100 mg/l und stabilisiert sich nach einer weiteren Woche.

Durch das erfindungsgemässe Verfahren werden den Zellen auf schonende Weise ausreichend Nährstoffe zugeführt sowie Stoffwechselprodukte abgeführt. Ein schädlicher physikalischer oder chemischer Einfluss kann durch die Membranen von den Zellen ferngehalten werden, und durch die diskontinuierliche schonende Betriebsweise wird die Scherungsbeanspruchung praktisch verhindert. Durch die Anordnung der Membrankammern in Stapeln ist eine hohe Volumenausnützung gegeben und damit die Möglichkeit, technisch relevante Kulturgrössen als Einheitsoperation zu erreichen.

**Patentansprüche**

1. Verfahren zum Kultivieren von humanen, tierischen pflanzlichen sowie hybriden Zellen und Mikroorganismen in einem Bioreaktor mit wenigstens drei aneinander anliegenden Kammern, wobei wenigstens eine erste Kammer eine Zellzuchtkammer (1), eine zweite Kammer eine Mediumkammer (2) und eine dritte Kammer eine Produktkammer (3) ist und die einzelnen Kammern voneinander durch Membranen (A, B) begrenzt sind, dadurch gekennzeichnet, dass die Zellen innerhalb der Zellzuchtkammer (1) an Trägern aus netzartigen Geweben (25, 25') immobilisiert werden, die die Membranen (A, B) mechanisch unterstützen und dass deren rahmenartige Ränder (27, 27', 28, 28') als Dichtung und gleichzeitig als Distanzkörper dienen, wobei eine mit einem sauerstoffhaltigen Gas angereicherte Nährlösung enthaltende Mediumkammer (2) in ihrer Längsrichtung kontinuierlich durchströmt wird und dass das Produkt aus der Zellzuchtkammer (1) nur diskontinuierlich abgezogen wird.

2. Vorrichtung zur Durchführung des Verfahrens nach Anspruch 1, dadurch gekennzeichnet, dass wenigstens eine Zellzuchtkammer (1) über eine, auf einem netzartigen Gewebe (25) abgestützte Membran (A) mit einer Mediumkammer (2) in Verbindung steht, dass die erste Membran (A) eine obere Ausschlussgrenze von 100'000 Dalton aufweist und das Produkt vom Nährmedium trennt, dass die Zellzuchtkammer (1) über eine zweite, auf einem anderen netzartigen Gewebe (25') abgestützte Membran (B) an die Produktkammer (3) angeschlossen ist, wobei die zweite Membran (B) eine Porosität aufweist, die den Abzug des Produktes aus der Zellzuchtkammer (1) unter Verhinderung des Abzugs der Zellen erlaubt.

3. Vorrichtung nach Anspruch 2, dadurch gekennzeichnet, dass die zweite Membran (B) eine Porengrösse zwischen 0,1 μm und 0,6 μm aufweist.

4. Vorrichtung nach Anspruch 2, dadurch gekennzeichnet, dass die Membranen (A, B) in der Zellzuchtkammer (1) einen Abstand von 0,3 bis 10 mm aufweisen.

5. Vorrichtung nach Anspruch 2, dadurch gekennzeichnet, dass eine Mess-Sonde (7) durch einen orthogonal durch die beiden an die Zellzuchtkammer (1) anliegenden Kammern (2; 3) hindurchführenden Anschlußstutzen (6') und eine weitere Mess-Sonde (7') durch einen Anschlußstutzen (6") mit dem Messkopf (7a) innerhalb der Zellzuchtkammer (1) fixiert ist.

6. Vorrichtung nach Anspruch 2, dadurch gekennzeichnet, dass die netzartigen Gewebe (25, 25') aus Fluorkohlenwasserstoffen bestehen.

7. Vorrichtung nach Anspruch 6, dadurch gekennzeichnet, dass die Fäden der netzartigen Gewebe (25, 25') mit ihren Rahmen spitze Winkeln einschliessen und dass die Rahmen (27, 27', 28, 28') gleichzeitig als Dichtungs- und Distanzierungskörper ausgebildet sind.

**Claims**

1. Process for the culture of human, animal, plant and hybrid cells and micro-organisms in a bioreactor having at least three adjacent chambers, whereby at least one first chamber is a cell culture chamber (1), a second chamber is a medium chamber (2) and a third chamber is a product chamber (3), and the individual chambers are delimited from one another by membranes (A, B), characterised in that the cells are immobilised within the cell culture chamber (1) on carriers of net-like tissues (25, 25') which mechanically support the membranes (A, B), and in that their frame-like edges (27, 27', 28, 28') serve as a seal and simultaneously as spacers, whereby a medium chamber (2) containing nutrient solution enriched with an oxygen-containing gas is continuously flowed through in its longitudinal direction, and in that the product is only drawn off from the cell culture chamber (1) discontinuously.

2. Apparatus for carrying out the process according to Claim 1, characterised in that at least one cell culture chamber (1) is connected to a medium chamber (2) via a membrane (A) supported on a net-like tissue (25), in that the first membrane (A) has an upper exclusion limit of 100'000 dalton and separates the product from the nutrient medium, in that the cell culture chamber (1) is connected to the product chamber (3) via a second membrane (B) supported on another net-like tissue (25'), whereby the second membrane (B) has a porosity which enables the product to be drawn off from the cell culture chamber (1) while preventing the cells from being drawn off.

3. Apparatus according to Claim 2, characterised in that the second membrane (B) has a pore size of between 0.1 μm and 0.6 μm.

4. Apparatus according to Claim 2, characterised in that the membranes (A, B) in the cell culture chamber (1) are at a distance of 0.3 to 10 mm.

5. Apparatus according to claim 2, characterised in that a measuring probe (7) is fixed by means of a connecting sleeve (6') passing orthogonally through the two chambers (2; 3) adjacent to the cell culture chamber (1) and a further measuring probe (7') is fixed by means of a connecting sleeve (6") with the measuring head (7a) within the cell culture chamber (1).

6. Apparatus according to Claim 2, characterised in that the net-like tissue (25, 25') comprises fluorohydrocarbons.

7. Apparatus according to Claim 6, characterised in that the threads of the net-like tissue (25, 25') form acute angles with their frame, and in that the frames (27, 27', 28, 28') are simultaneously constructed as sealing and spacer bodies.

**Revendications**

1. Procédé pour la culture de cellules humaines, animales, végétales ainsi que de cellules hybrides et de micro-organismes dans un bioréacteur avec au moins trois chambres contiguës, au moins une première chambre constituant une chambre de culture de cellules (1); une seconde chambre, une chambre de milieu (2) et une troisième chambre, une chambre de produit (3), et les différentes chambres étant séparées les unes des autres par des membranes (A, B), caractérisé en ce que, à l'intérieur de la chambre de culture de cellules (1), les cellules sont immobilisées sur des supports en tissus réticulés (25, 25') qui soutiennent les membranes (A, B) de manière mécanique, que leurs bords en forme de cadre (27, 27', 28, 28') servent à la fois de joint d'étanchéité et de pièce d'écartement, une chambre de milieu (2) contenant une solution nutritive enrichie d'un gaz oxygéné étant traversée en continu dans le sens de son extension longitudinale, et que le produit n'est évacué que de manière discontinue de la chambre de culture de cellules (1).

2. Dispositif pour la mise en oeuvre du procédé selon la revendication 1, caractérisé en ce qu'au moins une chambre de culture de cellules (1) communique avec une chambre de milieu (2) par l'intermédiaire d'une membrane (A) soutenue par un tissu réticulé (25), que la première membrane (A) présente une limite d'exclusion de 100'000 Daltons et sépare le produit du milieu nutritif, que la chambre de culture de cellules (1) est raccordée à la chambre de produit (3) par l'intermédiaire d'une seconde membrane (B) soutenue par un autre tissu réticulé (25'), la seconde membrane (B) présentant une porosité qui permet le soutirage du produit de la chambre de culture de cellules (1) en évitant l'évacuation des cellules.

3. Dispositif selon la revendication 2, caractérisé en ce que la seconde membrane (B) présente une grosseur de pores comprise entre 0,1 μm et 0,6 μm.

4. Dispositif selon la revendication 2, caractérisé en ce que les membranes (A, B) se trouvent dans la chambre de culture de cellules (1) à une distance de 0,3 à 10 mm l'une de l'autre.

5. Dispositif selon la revendication 2, caractérisé en ce qu'une sonde de mesure (7) est fixée au moyen d'un ajutage (6') traversant orthogonalement les deux chambres (2; 3) contiguës à la chambre de culture de cellules (1) et qu'une autre sonde de mesure (7') est fixée par un ajutage (6″) avec la tête de mesure (7a) placée à l'intérieur de la chambre de culture de cellules (1).

6. Dispositif selon la revendication 2, caractérisé en ce que les tissus réticulés (25, 25′) sont réalisés en hydrocarbures fluorés.

7. Dispositif selon la revendication 6, caractérisé en ce que les fils des tissus réticulés (25, 25′) renferment avec leurs cadres des angles aigus et que les cadres (27, 27′, 28, 28′) sont conformés à la fois en joints d'étanchéité et en éléments d'écartement.

FIG.1

# FIG. 2

# FIG. 3

# FIG. 4

# FIG.5

# FIG.6

EP 0 155 237 B1

FIG.7

7